# EUROPEAN PATENT SPECIFICATION

(11) **EP 0 423 419 B1**
(45) Date of publication and mention of the grant of the patent: **07.09.1994**
(21) Application number: 90107475.7
(22) Date of filing: 19.04.1990
(51) Int. Cl.: A61K 37/64, A61K 31/35, A61K 35/78

(54) **Inhibitive agent against activity of alpha-amylase**
Mittel zur Hemmung der alpha-Amylase Aktivität
Agent inhibiteur de l'activité de l'alpha-amylase

(30) Priority: 19.10.1989 JP 270228/89
(43) Date of publication of application: 24.04.1991
(73) Proprietor: MITSUI NORIN CO., LTD., Tokyo (JP)
(72) Inventor: Hara, Yukihiko, Fujieda-shi, Shizuoka-ken (JP); Honda, Miwa, Fujieda-shi, Shizuoka-ken (JP)
(74) Representative: Türk, Dietmar

(56) References cited:
- US-A- 4 613 672
- US-A- 4 673 530
- US-A- 4 840 966
- JOURNAL OF THE CHINESE AGRICULTURAL CHEMICAL SOCIETY vol. 27, no. 3, September 1989, TAIWAN pages 406 - 417; HA C.L. et al: "In vitro inhibition of trypsin and -amylase activities by tea."
- ARCHIVES OF ORAL BIOLOGY vol. 33, no. 11, 1988, Great Britain pages 845 - 846; KASHKET S. et al: "Inhibition of salivary amylase by water-soluble extracts of tea."
- AGRICULTURAL AND BIOLOGICAL CHEMISTRY vol. 54, no. 8, August 1990, JAPAN pages 1939 - 1945; HARA Y. et al: "The inhibition of -amylase by tea polyphenols. "

## Description

The present invention relates to a novel inhibitive agent against the activity of α-amylase or, more particularly, to an inhibitive agent against the activity of α-amylase with high specificity in the reaction with α-amylase.

A serious problem in these days called "an age of gluttony" is that many people suffer from corpulence and an adult disease or geriatric disease as a consequence of corpulence so that dieting or control of food intake is an important means for health control. In the midst of this current, dietary fibers, which cannot be absorbed as food, are highlighted and utilized in various aspects. The effect of dietary fibers consists in the control of the absorption of harmful substance including carcinogenic ones and enhancement of the evacuating performance of the intestines rather than positive suppression of corpulence.

α-Amylase is a kind of digestive enzymes capable of hydrolyzing polysaccharides and contained in the saliva and pancreatic juice of human. Accordingly, inhibition of the activity of α-amylase would hopefully have an effect to prevent corpulence with adequate satisfaction of the appetite and exhibit a therapeutic effect for diabetes. Several inhibitive agents against the activity of α-amylase have been developed with such an object although none of them is quite satisfactory in the activity with certain undesirable side effects in some of them.

Accordingly, the development of a novel inhibitive agent against activity of α-amylase which can be administered to patients without particular care to undesirable side effects against human body has been eagerly awaited.

In Archs. oral Biol. Vol. 33 (11), 1988, p. 845 - 846 the effects of tea extracts in reducing caries in humans as well as in animals are described. In such report the inhibition of salivary amylase activity by extracts of a commercial tea is demonstrated by experiments.

In J. Chinese Agric. Chem. Soc., 27 (3), 1989, p. 406 - 417 the in vitro inhibition of trypsin and α-amylase activities by tea is described. In such report it is suggested that the inhibition of trypsin and α-amylase was related to the polyphenols of tea.

An object of the present invention is to provide the use of an inhibitive agent against activity of α-amylase as mentioned above. The inventor has continued extensive investigations of natural products to discover a substance capable of exhibiting the desired effect without the problems which usually ensue in the use of chemically synthesized compounds.

Thus, the inhibitive agent against activity of α-amylase according to the present invention comprises tea as the medicinally effective ingredient.

Further, the inhibitive agent of the invention comprises polyphenol compounds in tea as the effective ingredient. The polyphenol compound in tea as the effective ingredient in the inhibitive agent is selected from the group consisting of epigallocatechin gallate, epicatechin gallate, epigallocatechin, epicatechin,

Thus, present invention relates to the use of a tea polyphenol, selected from the group consisting of epigallocatechin gallate and the isomer thereof, epicatechin gallate and the isomer thereof, free theaflavin, theaflavin monogallate A, theaflavin monogallate B and theaflavin digallate for inhibiting the activity of α-amylase.

Further, present invention relates to use of a tea polyphenol, selected from the group consisting of epigallocatechin gallate and the isomer thereof, epicatechin gallate and the isomer thereof, free theaflavin, monogallate A, theaflavin monogallate B and theaflavin digallate for the manufacture of a medicament for therapeutic treatment of corpulence.

The tea polyphenol compounds as the principal effective ingredients in the inventive inhibitive agent against activity of α-amylase the therapeutic treatment of corpulence include the tea catechin compounds represented by the general formula (I) given below and the theaflavin compounds represented by the general formula (II) given below: 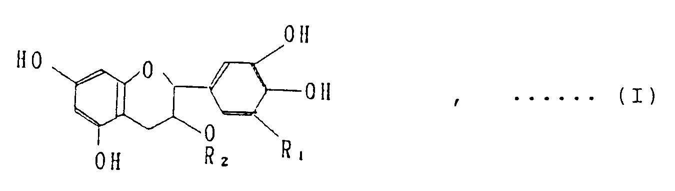
in which R₁ is a hydrogen atom or a hydroxy group and R₂ is a hydrogen atom or a 3,4,5-trihydroxy benzoyl group; and 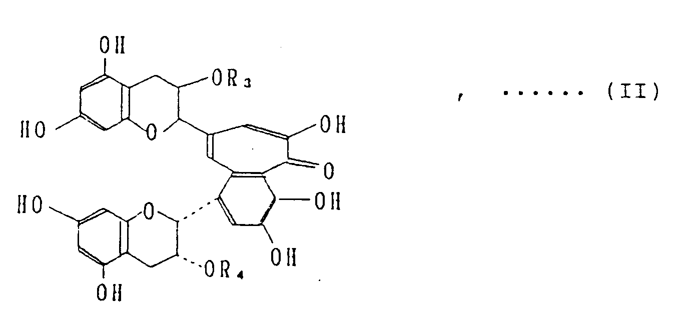
in which R₃ and R₄ are, each independently from the other, a hydrogen atom or a 3,4,5-trihydroxy benzoyl group.

Particular examples of the tea catechin compounds represented by the general formula (I) include: (-)epicatechin, which is a compound of the formula (I) with R₁ = H and R₂ = H; (-)epigallocatechin, which is a compound of the formula (I) with R₁ = OH and R₂ = H; (-)epicatechin gallate, which is a compound of the formula (I) with R₁ = H and R₂ = 3,4,5-trihydroxy benzoyl group; and (-)epigallocatechin gallate, which is a compound of the formula (I) with R₁ = OH and R₂ = 3,4,5-trihydroxy benzoyl group. Particular examples of the theaflavin compounds include: free theaflavin, which is a compound of the formula (II) with R₃ = H and R₄ = H; theaflavin monogallate A, which is a compound of the formula (II) with R₃ = 3,4,5-trihydroxy benzoyl group and R₄ = H; theaflavin monogallate B, which is a compound of the formula (II) with R₃ = H and R₄ = 3,4,5-trihydroxy benzoyl group; and theaflavin digallate, which is a compound of the formula (II) with R₃ = 3,4,5-trihydroxy benzoyl group and R₄ = 3,4,5-trihydroxy benzoyl group.

The above described tea polyphenol compounds can be prepared from tea leaves as the starting material and a method for the preparation thereof and a typical example of the product composition are described, for example, in US-A-4,613,672; US-A-4,673,530 and in Japanese Patent Kokai 61-130285 and elsewhere.

When the prepared inhibitive agent against activity of α-amylase for the treatment of corpulence is to be processed into a medicament form or an additive for food, the above described tea polyphenol as the effective ingredient as such is admired with the base without or with dilution with water or alcohol. The concentration thereof in the digestive tract is preferably in the range from 0.1 µM to 5 mM or, more preferably, from 0.5 µM to 1 mM.

The above described inhibitive agent against activity of α-amylase comprises, as the effective ingredient, a natural product which is drinkable taken in daily life in a considerably large volume so that it is absolutely free from the problem of undesirable side effects against human body not only when it is used as a medicine but also when it is used as an additive of food. Moreover, the effectiveness thereof is so high that activity of α-amylase can be effectively inhibited by the addition thereof even in a very low concentration to provide a means for inhibiting activity of α-amylase.

In the following, examples are given to illustrate the invention in more detail.

### Example 1

The enzyme used here was a product of α-amylase prepared from human saliva and supplied by Sigma Co.

A 150 µl of the enzyme solution (0.44 U/ml in a buffer solution) was added to 1230 µl of the sample solution and the mixture was incubated at 37°C for 10 minutes. Thereafter, the sample solution was admixed with 120 µl of a solution of soluble starch as the substrate so as to have a final concentration of the substrate of 2.0 mg/ml to effect the reaction at 37°C. A 200 µl aliquot of the solution was taken in every 3 minutes from the solution while the reaction was proceeding and the reducing sugar produced therein was determined by the measurement of the absorbance at a wave-length of 540 nm according to the method of Bernfeld described in Meth. Enzymol., volume 1, page 49 (1959) by P. Bernfeld. The value of the absorbance was converted by calculation into the amount of maltose present and from this the reaction velocity was calculated according to the conventional procedure. The concentration of the solution for 50% inhibition of the activity of α-amylase was determined with each sample assuming that the activity of α-amylase was 100% when the reaction velocity was equal to that in the control in which the same volume of the buffer solution was added in place of the sample solution. The results are shown in Table 1 below.

**Table 1**

| Sample | Concentration for 50% inhibition |
|---|---|
| Gallic acid | » 1mM |
| Epicatechin | » 1mM |
| Isomer of epicatechin | » 1mM |
| Epicagallcatechin | » 1mM |
| Isomer of epigallocatechin | » 1mM |
| Epicatechin gallate | 130 µM |
| Isomer of epicatechin gallate | 20 µM |
| Epigallocatechin gallate | 260 µM |
| Isomer of epigallocatechin gallate | 55 µM |
| Free theaflavin | 18 µM |
| Theaflavin monogallate A | 1.0 µM |
| Theaflavin monogallate B | 1.7 µM |
| Theaflavin digallate | 0.6 µM |

The conclusion that could be derived from the above results is that, among the catechin compounds shown in the table, epicatechin, epigallocatechin and isomers thereof have almost no power for the inhibition of the activity of α-amylase but the other catechin compounds and theaflavin compounds have strong power for the inhibition of the activity of α-amylase.

### Example 2

Each of the 12-weeks old male rats of the Wistar strain, divided into a test group and a control group, was fed a high-carbohydrate diet either with or without respectively 1% by weight of Polyphenon® 100 which was a crude mixture of catechin compounds in a proportion shown in Table 2 below.

**Table 2**

| Catechin compound | Polyphenon 100 | |
|---|---|---|
| | Content, % | Relative content, % |
| Gallocatechin | 1.44 | 1.6 |
| Epigallocatechin | 17.57 | 19.3 |
| Catechin | - | - |
| Epicatechin | 5.81 | 6.4 |
| Epigallocatechin gallate | 53.90 | 59.1 |
| Epicatechin gallate | 12.51 | 13.7 |
| Total | 9̅1̅.̅2̅3̅ | 1̅0̅0̅ |

The formulation of the high-carbohydrate diet given to the control animals was as shown below in Table 3. In the diet given to the test animals, the formulation was modified by decreasing the amount of the starch powder to 70.0% and addition of 1.0% of Polyphenon® 100 instead.

**Table 3**

| Constituent | Content in high-carbohydrate diet (control), % | Content in the diet being added Polyphenon 100, % |
|---|---|---|
| Casein | 22.0 | 22.0 |
| Salt mix | 4.0 | 4.0 |
| Corn oil | 2.0 | 2.0 |
| Vitamin complex | 1.0 | 1.0 |
| Starch powder | 71.0 | 70.0 |
| Polyphenone® 100 | - | 1.0 |
| Total | 1̅0̅0̅ | 1̅0̅0̅ |

After 7 days of raising in this manner, the feces discharged from each animal were collected for one day and weighed to examine the change in the amount thereof caused by the addition of Polyphenon® 100 to the diet. The results were that the amount in the control animals was 1.01 g per day per animal while the amount in the test animals was 1.78 g per day per animal to support the conclusion that the addition of the catechin compounds to the diet was effective to increase the amount of feces discharge. This result means that the catechin compounds act in a similar manner to dietary fibers in promoting the evacuating performance of the intestines by decreasing absorption of the carbohydrates as a consequence of the power to inhibit the activity of amylase.

### Example 3

When the inhibitive agent against the activity of α-amylase is administrated to human body, the dose to be taken orally is 0.1 to 10 g per day or, preferably, 2 to 5 g per day. The form of the medicament is not particularly limitative and it can be taken as such or in for example the form of a powder, tablet or capsule, optionally, with admixture of an extending agent. When the inventive agent is used as an additive in food, it is added to various kinds of processed food and confectionery such as breads, noodles, cakes, biscuits and cookies in an amount of 0.2 to 1.0% by weight.

### Example 4

An animal test was conducted by using ICR mice as the test animals to examine the acute toxicity of the inventive inhibitive agent against the activity of α-amylase. The values of LD₅₀ calculated according to the Van der Waerdrn method within the confidence limit were: 2412 mg/kg in the oral administration of the same crude mixture of catechin compounds as used in Example 2; 55.2 mg/kg in the intraperitoneal administration of a crude mixture of theaflavin compounds of the composition shown in Table 5 below; and 150 mg/kg in the intraperitoneal administration of epigallocatechin gallate.

**Table 5**

| Compound | Content, %, in the crude mixture of theaflavin compounds |
|---|---|
| Free theaflavin | 10.0 |
| Theaflavin monogallate A | 22.3 |
| Theaflavin monogallate B | 19.5 |
| Theaflavin digallate | 32.5 |
| (+) Catechin | 0.3 |
| (-) Epicatechin | 1.8 |
| (-) Epigallocatechin gallate | 4.7 |
| Isomer of (-) epigallocatechin gallate | 1.0 |
| (-) Epicatechin gallate | 3.9 |
| Others (isomers of theaflavin, etc.) | 4.0 |

## Claims

1. Use of a tea polyphenol, selected from the group consisting of epigallocatechin gallate and the isomer thereof, epicatechin gallate and the isomer thereof, free theaflavin, theaflavin monogallate A, theaflavin monogallate B and theaflavin digallate for inhibiting the activity of α-amylase.

2. Use of a tea polyphenol, selected from the group consisting of epigallocatechin gallate and the isomer thereof, epicatechin gallate and the isomer thereof, free theaflavin, theaflavin monogallate A, theaflavin monogallate B and theaflavin digallate for the manufacture of a medicament for therapeutic treatment of corpulence.

## Patentansprüche

1. Verwendung eines Tee-Polyphenols, ausgewählt aus der Gruppe, die besteht aus Epigallocatechingallat und seinem Isomer, Epicatechingallat und seinem Isomer, freiem Theaflavin, Theaflavinmonogallat A, Theaflavinmonogallat B und Theaflavindigallat, zur Inhibierung der Aktivität von α-Amylase.

2. Verwendung eines Tee-Polyphenols, ausgewählt aus der Gruppe, die besteht aus Epigallocatechingallat und seinem Isomer, Epicatechingallat und seinem Isomer, freiem Theaflavin, Theaflavinmonogallat A, Theaflavinmonogallat B und Theaflavindigallat, zur Herstellung eines Arzneimittels für die therapeutische Behandlung der Korpulenz.

## Revendications

1. Utilisation d'un polyphénol extrait du thé choisi parmi le groupe constitué de l'épigallocatéchine gallate et son isomère, l'épicatéchine gallate et son isomère, la théaflavine libre, la théaflavine monogallate A, la théaflavine monogallate B et la théaflavine digallate pour une inhibition de l'activité de l'α-amylase.

2. Utilisation d'un polyphénol extrait du thé choisi parmi le groupe constitué de l'épigallocatéchine gallate et son isomère, l'épicatéchine gallate et son isomère, la théaflavine libre, la théaflavine monogallate A, la théaflavine monogallate B et la théaflavine digallate pour la préparation d'un médicament destiné au traitement thérapeutique de la corpulence.
